# EUROPEAN PATENT SPECIFICATION

(11) **EP 2 931 130 B1**
(45) Date of publication and mention of the grant of the patent: **13.10.2021**
(21) Application number: 13861861.6
(22) Date of filing: 11.12.2013
(51) Int. Cl.: A61B 8/12

(54) **ROTATIONAL SENSING CATHETER WITH SELF-SUPPORTING DRIVE SHAFT SECTION**
ROTIERENDER MESSKATHETER MIT SELBSTTRAGENDEM ANTRIEBSWELLENABSCHNITT
CATHÉTER DE DÉTECTION DE ROTATION AVEC SECTION DE TIGE D'ENTRAÎNEMENT AUTOPORTEUSE

(30) Priority: 13.12.2012 US 201261736588 P
(43) Date of publication of application: 21.10.2015
(73) Proprietor: Philips Image Guided Therapy Corporation, San Diego CA 92130 (US)
(72) Inventor: VAN HOVEN, Dylan, Oceanside, California 92057 (US)
(74) Representative: de Haan, Poul Erik
(86) International application number: PCT/US2013/074344
(87) International publication number: WO 2014/093465

(56) References cited:
- WO-A1-01/89621
- WO-A1-93/16642
- WO-A1-2007/059281
- WO-A2-01/34240
- JP-A- 2012 152 353
- US-A1- 2004 073 203
- US-A1- 2008 167 602
- US-A1- 2009 156 941
- US-A1- 2009 163 818
- US-A1- 2010 234 736
- US-A1- 2010 318 064
- US-A1- 2011 098 573

## Description

### TECHNICAL FIELD

An embodiment of the present disclosure relates generally to the field of medical devices and, more particularly, to catheter apparatus used in internal vasculature diagnostic procedures.

### BACKGROUND

Various techniques and systems have recently been developed to visualize the anatomy of vascular occlusions by using intravascular ultrasound (IVUS) imaging. IVUS techniques are catheter based and provide a real-time sectional image of the arterial lumen and the arterial wall. An IVUS catheter includes one or more ultrasound transducers at the distal tip of the catheter by which images containing cross-sectional information of the artery under investigation can be determined. IVUS imaging permits visualization of the configuration of the obstructing material and, in varying degrees, the boundaries of the intimal and medial layers of the arterial wall.

One common type of IVUS imaging catheter system typically includes an arrangement in which a single transducer at the distal end of the catheter is rotated at high speed (up to about 2000 rpm) to generate a rapid series of 360-degree ultrasound sweeps. Such speeds result in generation of up to about thirty images per second, effectively presenting a real-time image of the diseased artery.

The transducer is mounted on the end of a drive shaft or cable that is connected to a motor drive at the proximal end of the catheter. The rotating transducer is housed within a sheath that does not interfere with the ultrasound and protects the artery from the rapidly spinning drive shaft. Thus, an IVUS imaging (or "sensing") catheter may be advanced to the region of an occlusion using conventional angiographic techniques and then may be operated to provide real-time sectional images of the vascular lumen in the arterial wall, including the occluding material and intimal and medial layers of the artery wall. Other types of catheter-based systems for use in visualizing the internal anatomy of body portions implementing sheath-enclosed movable sensing/imaging elements disposed on elongated drive shaft structures arc also known, including photo-acoustic, optical coherence tomography, phased array/multiple transducer, and spectroscopic systems.

Medical sensing catheters of these representative types comprise a tubing assembly through which the drive cable movably extends, the tubing assembly typically including a sheath insertable into the patient and having a proximal end fixed to a telescope section which permits the drive cable, and thus the sensor, to be selectively moved though the patient's body via the interior of the inserted sheath which remains stationary in the patient's body. The telescope section comprises a tubular outer catheter or telescope member, to the distal end of which the proximal end of the sheath is anchored. The telescope section also has a tubular inner catheter or telescope member which telescopes into the interior of the outer telescope member through its proximal end and is movable through the interior of the outer telescope member between retracted and extended positions relative to the outer telescope member. The drive cable is secured to the inner telescope member for longitudinal movement therewith relative to the outer catheter member.

Distal movement of the inner telescope member toward its retracted position distally pushes the drive cable and the sensor through the sheath, and proximal movement of the inner telescope member toward its extended position pulls the drive cable and the sensor back through the sheath. When the inner telescope member is moved to its extended position a portion of the drive cable extending through the interior of the outer catheter member between the distal end of the outer telescope member and the distal end of the inner catheter member is substantially unsupported and unconstrained within the telescope section.

In response to a subsequent movement of the inner telescope member distally toward its retracted position the exposed, unsupported portion of the drive cable may undesirably be caused to buckle within the telescope section, thereby hindering a desired distal advancement of the drive cable through the sheath and potentially damaging the cable. A previously proposed solution to this potential drive cable buckling problem has been to position a separate reinforcing structure within the telescope section to support the portion of the drive cable extending through the telescope section when the inner telescope member is moved proximally away from its retracted position.

This previously proposed drive cable supporting technique, however, has proven to be less that wholly satisfactory because it requires the provision and installation in the overall catheter assembly of at least one additional component to support the otherwise unsupported section of the drive cable within the telescope section, thereby undesirably increasing the catheter assembly cost, complexity and manufacturing time. As may be readily seen from the foregoing, a need exists for an improved solution to the above-described catheter drive cable buckling problem. It is to this need that the present invention is primarily directed.

US 2009/163818 discloses a low profile catheter comprising a catheter sheath, a short guidewire receiver attached to the distal end of the catheter sheath, and a telescope assembly at the proximal end. The catheter sheath comprises a main portion and a tapered portion for increased flexibility toward the distal end of the catheter. In one embodiment, a rotatable and translatable imaging core is received within the catheter sheath for ultrasound imaging. In an embodiment, the catheter sheath extends through a portion of the telescope assembly to provide enhanced support of the imaging core within the telescope assembly.

US 2011/098573 relates to a catheter assembly for an intravascular ultrasound system, the assembly including an ultrasound transducer disposed in an image device housing within a lumen of a catheter. The ultrasound transducer transforms applied electrical signals to acoustic signals within a frequency bandwidth centered at an operational frequency and having variable electrical impedances over one or more frequencies within the bandwidth. A distal drive cable is coupled to the imaging device housing. A connector housing couples the distal drive cable to a proximal drive cable.

### BRIEF DESCRIPTION OF THE DRAWINGS

FIG. 1 is an enlarged scale longitudinally foreshortened schematic cross-sectional view through medical sensing catheter apparatus embodying principles of the present invention;
FIG. 2 is a longitudinally foreshortened schematic cross-sectional view through a telescope section of the catheter apparatus with an inner telescope portion of the section being in its fully retracted position; and
FIG. 3 is a view similar to that in FIG. 2 but with the inner telescope portion of the telescope section being in its fully extended position.

### DETAILED DESCRIPTION

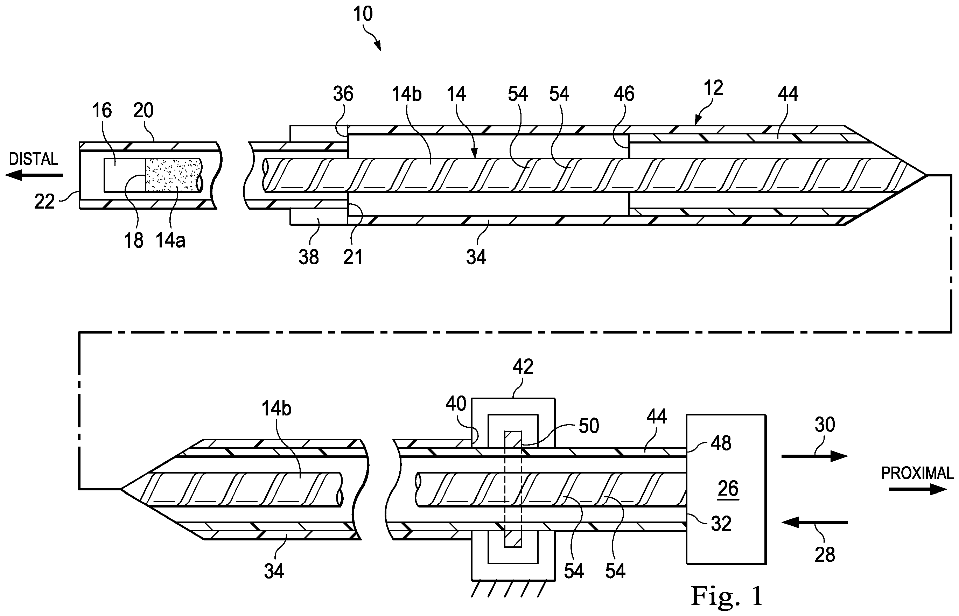

A catheter 10 embodying principles of the present invention is schematically depicted in FIGS. 1-3. By way of non-limiting example, the catheter apparatus 10 is a medical sensing catheter, and more specifically is an intravascular ultrasound (IVUS) imaging catheter. Catheter 10 includes an elongated flexible tubular assembly 12 that circumscribes an elongated flexible drive shaft or cable 14 having an ultrasound sensor 16 on its distal end 18.

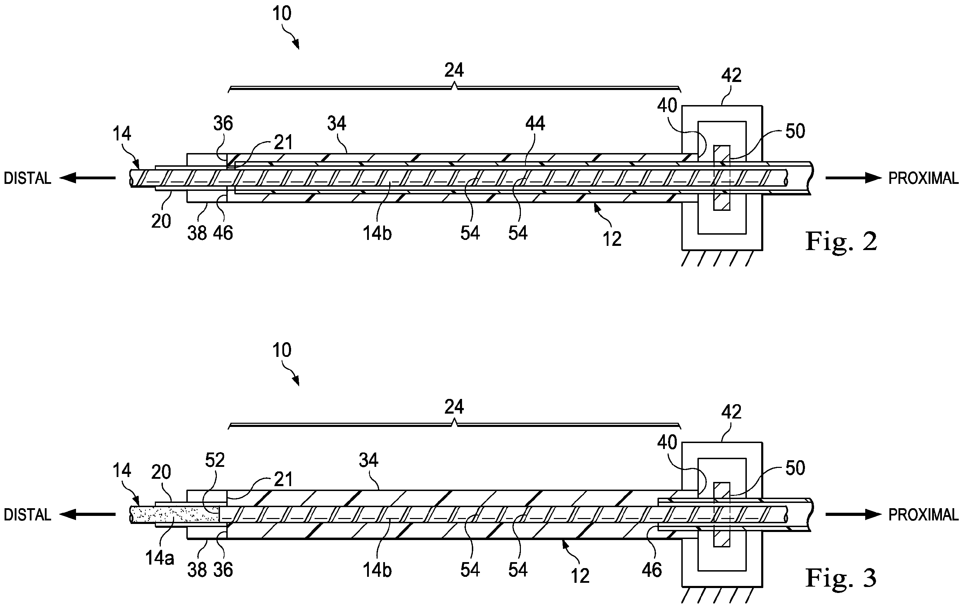

The tubular assembly 12 that circumscribes the drive cable 14 and the sensor 16 includes a sheath 20 having a proximal end 21, and a distal end 22 insertable into the body of a patient, and a telescope section 24 (see FIGS. 2 and 3) that facilitates movement of the drive cable 14 distally and proximally through the sheath 20 while it remains stationary within the patient's body. Selective rotation and translation of the drive cable 14 relative to the sheath 20 is effected by a conventional, schematically depicted translational/rotational drive mechanism 26 (FIG. 1) that may be selectively translated in distal and proximal directions as respectively illustrated by arrows 28,30 in FIG. 1. The drive mechanism 26 is operatively coupled to the proximal end 32 of the drive cable 14 and functions in a conventional manner to translate and rotate the drive cable 14.

Telescope section 24 includes an elongated flexible tubular outer catheter or telescope member 34 having a distal end 36 fixedly secured to an annular coupling 38 that circumscribes and is fixedly secured to the proximal end of the sheath 20. The proximal end 40 of the outer telescope member 34 is anchored to a schematically depicted stationary support structure 42 distally positioned relative to the drive mechanism 26. The telescope section 24 further includes an elongated flexible tubular inner catheter or telescope member 44 which has distal and proximal ends 46,48 (see FIG. 1). Proximal end 48 is secured to the drive mechanism 26, and the inner telescope member 44 slidably extends through an O-ring seal member 50 carried by the stationary support structure 42 which may be of a conventional construction and may be assembled around the O-ring 50.

According to a feature of the present invention the O-ring seal 50 is formed of a self-lubricating material, representatively a fluoroelastomeric material. The use of a self-lubricating seal member substantially facilitates and quickens the assembly of the support structure 42 by eliminating the necessity of lubricating the seal and one or more of the support structure parts prior to using the support structure 42.

As shown in FIGS. 1-3, the inner telescope member 44 is distally telescoped into the outer telescope member portion 34 of the overall tubular assembly 12 for translation relative thereto (by means of the drive mechanism 26) between a retracted position shown in FIG. 2 (in which the sensor 16 is distally advanced within the sheath 20) and an extended position shown in FIG. 3 (in which the sensor is proximally retracted within the sheath 20).

According to a further feature of the present invention, the flexible drive shaft 14 is not of a uniform construction along its length. Instead, a first portion 14a of the drive shaft 14 extending proximally away from the sensor 16 (see FIGS. 1 and 3) is of a conventional construction, representatively of a helically wound wire construction. Fixedly and coaxially secured to the proximal end of the drive shaft portion 14a (as, for example, by an end weld 52 as shown in FIG. 3), and extending proximally away therefrom, is a second representatively metal drive shaft portion 14b. The relative lengths of the drive shaft sections 14a,14b are sized in a manner such that when the inner telescope member 44 is in its FIG. 3 extended position the section 14b extends from the section 14a at least through essentially the entire interior length of the telescope section 24.

The flexible drive shaft section 14b has a stiffness sufficiently greater than that of the drive shaft portion 14a so as to be self-supporting during operation within the telescope section 24 when, as depicted in FIG. 3, the inner telescope member 44 is proximally moved away from its FIG. 2 retracted position toward or completely to its FIG. 3 extended position. By way of non-limiting example, the flexible drive shaft section 14b may be a tubular helically cut metal beam member with the representatively illustrated helical cut patterns 54 formed on its exterior surface. Although the illustrated embodiment shows the drive shaft section 14b as straight, it will be appreciated that the helical cuts 54 along the tubular metal beam allow the drive shaft section 14b to bend, if necessary, during operation and still rotate the sensor 16. However, the drive shaft section 14b could alternatively be of a variety of other materials and constructions without departing from principles of the present invention. For example, a sufficiently rigid polymer tube may be selected as the drive shaft section 14b that can be joined to the shaft section 14a via a mechanical coupling.

For purposes of manufacturing efficiency, the relatively stiffer self-supporting flexible drive shaft section 14b may, as schematically depicted in FIG. 1, extend from its connection 52 at the drive shaft section 14a (see FIG. 3) to the drive mechanism 26. Alternatively, the length of the drive shaft section 14b may be somewhat shorter and connected at its proximal end to a terminal drive shaft section of a different construction such as, for example, the helically wound wire material used in the drive shaft section 14a, or a solid metal material.

The unique incorporation in the catheter 10 of the self-supporting flexible drive shaft section 14b desirably eliminates the previous necessity of shielding and supporting a drive shaft portion exposed within the telescope section by providing and installing a separate protective structure within the telescope section.

While the catheter 10 has been representatively illustrated as being an IVUS catheter, it will be readily appreciated by those of ordinary skill in this particular art that other types of catheter structures with flexible internal drive shafts or cables and associated telescope sections may advantageously incorporate the above-described type of self-supporting cable structure without departing from principles of the present invention. Such other types of catheter structures and sensing elements include, for example, photo-acoustic, optical coherence tomography (OCT), phased array/multiple transducer, and spectroscopic systems. Still further, while the outer telescope member 34 is shown fixed to the proximal end 21 of the sheath 20, and the inner telescope member 44 is fixed to the drive mechanism 26, these fixation locations of the inner and outer telescope members 44,34 may be reversed such that the drive shaft 14 moves with the outer telescope member 34.

## Claims

1. A catheter apparatus (10) comprising:
a telescope section (24) including telescoped inner (44) and outer (34) tubular catheter members;
a sheath (20) having a proximal end (21) anchored to a distal end of one of said outer or inner tubular catheter member; and
an elongated flexible drive member (14) coaxially extending through said outer tubular catheter member and being proximally and distally movable through said sheath, said elongated flexible drive member having a distal portion (14a) disposed within said sheath, and a second portion (14b) extending proximally beyond said sheath and having a stiffness greater than that of said distal portion of said elongated flexible drive member so as to be self-supporting during operation within the telescope section when the inner tubular catheter member (44) is proximally moved away from a retracted position toward or completely to an extended position.

2. The catheter apparatus of claim 1 wherein said distal portion of said elongated flexible drive member is of a helically wound wire construction.

3. The catheter apparatus of claim 1 or claim 2 wherein said second portion of said elongated flexible drive member is of a helically cut metal beam construction.

4. The catheter apparatus of claim 3 wherein said distal and second portions of said elongated flexible drive member are end-welded (52) to one another.

5. The catheter apparatus of Claim 1 wherein said catheter apparatus is a medical sensing catheter including a sensing element secured to said distal portion of said elongated flexible drive member.

6. The catheter apparatus of claim 5 wherein said medical sensing catheter is an IVUS catheter, and said sensing element is a rotatable ultrasonic sensing element (16).

7. The catheter apparatus of Claim 1 wherein a portion of said inner tubular catheter member is slidingly supported within a self-lubricating O-ring seal member (50).

8. The catheter apparatus of claim 7 wherein said outer tubular catheter member has a proximal end (40), and said self-lubricating O-ring seal member is of a fluoroelastomeric material and is disposed proximally of said proximal end of said outer tubular catheter member.

9. The catheter apparatus of claim 4 wherein said sensing element is one of a photo-acoustic sensing element, an optical coherence tomography sensing element, a phased array/multiple transducer sensing element, and a spectroscopic system sensing element.

10. The catheter apparatus of claim 8 wherein said sheath and said flexible drive member are constructed and configured in a manner permitting operational rotation of said flexible drive member relative to said sheath at speeds up to about 2000rpm.

11. The catheter apparatus of Claim 1 wherein the outer tubular catheter member is fixed to the proximal end of the sheath, and the inner tubular catheter member is fixed to a drive mechanism (26) for selective rotation and translation of the elongated flexible drive member relative to the sheath.

12. The catheter apparatus of Claim 1 wherein the inner tubular catheter member is fixed to the proximal end of the sheath, and the outer tubular catheter member is fixed to a drive mechanism (26) for selective rotation and translation of the elongated flexible drive member relative to the sheath.

## Patentansprüche

1. Kathetervorrichtung (10), umfassend:
einen Teleskopabschnitt (24), der ein inneres (44) und ein äußeres (34) röhrenförmiges teleskopisches Katheterelement aufweist;
eine Hülse (20) mit einem proximalen Ende (21), das an einem distalen Ende des äußeren oder inneren röhrenförmigen Katheterelements verankert ist; und
ein längliches flexibles Antriebselement (14), das sich koaxial durch das äußere röhrenförmige Katheterelement erstreckt und proximal und distal durch die Hülse bewegbar ist, wobei das längliche flexible Antriebselement einen in der Hülse angeordneten distalen Abschnitt (14a) und einen zweiten Abschnitt (14b) aufweist, der sich proximal über die Hülse hinaus erstreckt und eine Steifigkeit aufweist, die größer ist als die des distalen Abschnitts des länglichen flexiblen Antriebselements, so dass er sich während des Betriebs innerhalb des Teleskopabschnitts selbst trägt, wenn das innere röhrenförmige Katheterelement (44) proximal aus einer eingefahrenen Position in Richtung auf oder vollständig in eine ausgefahrene Position bewegt wird.

2. Kathetervorrichtung nach Anspruch 1, wobei der distale Abschnitt des länglichen flexiblen Antriebselements von einer spiralförmig gewundenen Drahtkonstruktion gebildet wird.

3. Kathetervorrichtung nach Anspruch 1 oder Anspruch 2, wobei der zweite Abschnitt des länglichen flexiblen Antriebselements von einer spiralförmig geschnittenen Metallstangenkonstruktion gebildet wird.

4. Kathetervorrichtung nach Anspruch 3, wobei der distale und der zweite Abschnitt des länglichen flexiblen Antriebselements an ihren Enden miteinander verschweißt sind (52).

5. Kathetervorrichtung nach Anspruch 1, wobei die Kathetervorrichtung ein medizinischer Abtastkatheter ist, der ein Abtastelement aufweist, das an dem distalen Abschnitt des länglichen flexiblen Antriebselements gesichert ist.

6. Kathetervorrichtung nach Anspruch 5, wobei der medizinische Abtastkatheter ein IVUS-Katheter ist und das Abtastelement ein drehbares Ultraschallabtastelement (16) ist.

7. Kathetervorrichtung nach Anspruch 1, wobei ein Abschnitt des inneren röhrenförmigen Katheterelements verschiebbar innerhalb eines selbstschmierenden 0-Ring-Dichtelements (50) gelagert ist.

8. Kathetervorrichtung nach Anspruch 7, wobei das äußere röhrenförmige Katheterelement ein proximales Ende (40) aufweist und das selbstschmierende O-Ring-Dichtelement aus einem fluorelastomeren Material gebildet ist und proximal zu dem proximalen Ende des äußeren röhrenförmigen Katheterelements angeordnet ist.

9. Kathetervorrichtung nach Anspruch 4, wobei das Abtastelement entweder ein photo-akustisches Element, ein optisches Kohärenztomographie-Abtastelement, ein Abtastelement eines phasengesteuerten/multiplen Messwandlers oder ein Abtastelement eines spektroskopischen Systems ist.

10. Kathetervorrichtung nach Anspruch 8, wobei die Hülse und das flexible Antriebselement auf eine Weise aufgebaut und konfiguriert sind, die eine betriebsmäßige Drehung des flexiblen Antriebselements in Bezug auf die Hülse mit Drehzahlen von bis zu etwa 2000 UpM zulässt.

11. Kathetervorrichtung nach Anspruch 1, wobei das äußere röhrenförmige Katheterelement an dem proximalen Ende der Hülse fixiert ist und das innere röhrenförmige Katheterelement für eine selektive Drehung und Verschiebung des länglichen flexiblen Antriebselements in Bezug auf die Hülse an einem Antriebsmechanismus (26) fixiert ist.

12. Kathetervorrichtung nach Anspruch 1, wobei das innere röhrenförmige Katheterelement an dem proximalen Ende der Hülse fixiert ist und das äußere röhrenförmige Katheterelement für eine selektive Drehung und Verschiebung des länglichen flexiblen Antriebselements in Bezug auf die Hülse an einem Antriebsmechanismus (26) fixiert ist.

## Revendications

1. Appareil cathéter (10) comprenant:
une section télescopique (24) comprenant des éléments tubulaires télescopiques de cathéter intérieur (44) et extérieur (34);
une gaine (20) comportant une extrémité proximale (21) ancrée à une extrémité distale de l'un desdits éléments tubulaires de cathéter intérieur ou extérieur; et
un élément d'entraînement (14) flexible allongé s'étendant coaxialement à travers ledit élément tubulaire de cathéter externe et étant mobile de manière proximale et distale à travers ladite gaine, ledit élément d'entraînement flexible allongé comportant une partie distale (14a) disposée à l'intérieur de ladite gaine, et une seconde partie (14b) s'étendant de manière proximale au-delà de ladite gaine et présentant une rigidité supérieure à celle de ladite partie distale dudit élément d'entraînement flexible allongé de manière à être autoportant lors du fonctionnement à l'intérieur de la section télescopique lorsque l'élément tubulaire de cathéter interne (44) est déplacé de manière proximale d'une position rétractée vers ou complètement jusqu'à une position étendue.

2. Appareil cathéter selon la revendication 1, dans lequel ladite partie distale dudit élément d'entraînement flexible allongé est conçue sous la forme d'un fil enroulé en hélice.

3. Appareil cathéter selon la revendication 1 ou la revendication 2, dans lequel ladite seconde partie dudit élément d'entraînement flexible allongé est conçue sous la forme d'une poutre métallique découpée en hélice.

4. Appareil cathéter selon la revendication 3, dans lequel lesdites parties distale et seconde dudit élément d'entraînement flexible allongé sont soudées bout à bout (52) l'une à l'autre.

5. Appareil cathéter selon la revendication 1, dans lequel ledit appareil cathéter est un cathéter de détection médical comprenant un élément de détection fixé à ladite partie distale dudit élément d'entraînement flexible allongé.

6. Appareil cathéter selon la revendication 5, dans lequel ledit cathéter de détection médical est un cathéter IVUS, et ledit élément de détection est un élément de détection à ultrasons (16) rotatif.

7. Appareil cathéter selon la revendication 1, dans lequel une partie dudit élément tubulaire de cathéter interne est supportée de manière coulissante à l'intérieur d'un élément d'étanchéité (50) à joint torique autolubrifiant.

8. Appareil cathéter selon la revendication 7, dans lequel ledit élément tubulaire de cathéter externe comporte une extrémité proximale (40), et ledit élément d'étanchéité à joint torique autolubrifiant est en une matière fluoroélastomérique et est disposé à proximité de ladite extrémité proximale dudit élément de cathéter tubulaire externe.

9. Appareil cathéter selon la revendication 4, dans lequel ledit élément de détection est un élément de détection photo-acoustique et/ou un élément de détection de tomographie par cohérence optique et/ou un élément de détection à réseau phasé / transducteur multiple et/ou un élément de détection de système spectroscopique.

10. Appareil à cathéter selon la revendication 8, dans lequel ladite gaine et ledit élément d'entraînement flexible sont construits et conçus d'une manière permettant une rotation fonctionnelle dudit élément d'entraînement flexible par rapport à ladite gaine à des vitesses allant jusqu'à environ 2000 tr/min.

11. Appareil cathéter selon la revendication 1, dans lequel l'élément tubulaire de cathéter externe est fixé à l'extrémité proximale de la gaine, et l'élément tubulaire de cathéter interne est fixé à un mécanisme d'entraînement (26) pour sélectivement une rotation et une translation de l'élément d'entraînement flexible allongé par rapport à la gaine.

12. Appareil cathéter selon la revendication 1, dans lequel l'élément tubulaire de cathéter interne est fixé à l'extrémité proximale de la gaine, et l'élément tubulaire de cathéter externe est fixé à un mécanisme d'entraînement (26) pour sélectivement une rotation et une translation de l'élément d'entraînement flexible allongé par rapport à la gaine.
